# EUROPÄISCHE PATENTSCHRIFT

(11) **EP 1 655 279 B1**
(45) Veröffentlichungstag und Bekanntmachung des Hinweises auf die Patenterteilung: **22.02.2017**
(21) Anmeldenummer: 05023388.1
(22) Anmeldetag: 26.10.2005
(51) Int. Cl.: C07C 67/055, C07C 69/15, C07C 7/20, C07C 11/04

(54) **Verfahren zur Herstellung von Vinylacetat**
Process for the preparation of vinyl acetate
Procédé de préparation d'acétate de vinyle

(30) Priorität: 04.11.2004 DE 102004053184
(43) Veröffentlichungstag der Anmeldung: 10.05.2006
(73) Patentinhaber: Celanese Sales Germany GmbH, 65843 Sulzbach (DE)
(72) Erfinder: Rinne, Bernd, 65929 Frankfurt (DE); Hess, Stefan, Dr., 64521 Groß-Gerau (DE); Stamm, Johann, 65929 Frankfurt (DE); Nuber, Berthold, Dr., 65933 Frankfurt (DE)
(74) Vertreter: Kador & Partner

(56) Entgegenhaltungen:
- DE-A1- 2 359 286
- US-B1- 6 420 595

## Beschreibung

Die vorliegende Erfindung betrifft ein Verfahren zur Herstellung von Vinylacetat in der Gasphase aus Ethylen und Essigsäure in Gegenwart von Sauerstoff oder Sauerstoff enthaltenden Gasen unter Sättigung des den Vinylacetatreaktor zugeführten Reaktionsgemisches mit Essigsäure.

Es ist bekannt, dass man in der Gasphase Essigsäure mit Ethylen und Sauerstoff oder Sauerstoff enthaltenden Gasen bei erhöhtem Druck und erhöhter Temperatur an Palladium haltigen Festbettkatalysatoren zu Vinylacetat umsetzt. Als wirksame Katalysatoren haben sich Trägerkatalysatoren erwiesen, die neben Palladium auch noch Gold und Alkaliacetate als Promotoren enthalten.

So offenbart US-A-4,048,096 ein Verfahren zur Herstellung von Palladium, Gold und Kaliumacetat enthaltenden Katalysatoren, in dem zunächst das Trägermaterial mit einer wässrigen Lösung, die eine Mischung aus Palladium- und Goldsalzen enthält, imprägniert wird. Dabei entspricht das Volumen der Imprägnierlösung dem Porenvolumen des Trägermaterials. Dem Imprägnierschritt folgt eine Behandlung mit Alkaliverbindungen, beispielsweise mit einer wässrigen Natriummetasilikatlösung, wodurch die Metallsalze in wasserunlösliche Verbindungen überführt und auf dem Trägermaterial fixiert werden. Durch nachfolgende Behandlung mit einem Reduktionsmittel werden die Palladium- und Goldverbindungen zu den entsprechenden Metallen reduziert. Danach schließt sich eine Imprägnierung mit einer wässrigen Alkalimetallacetatlösung und ein abschließender Trocknungsschritt an. Man erhält einen Katalysator mit einer Schalenstruktur.

Gemäß US-A-5,332,710 wird der mit Palladium- und Goldsalzen imprägnierte Träger in eine Natrium- oder Kaliumhydroxid enthaltende wässrige Fixierlösung eingetaucht und darin wenigstens eine halbe Stunde lang bewegt. Dabei wird der mit Fixierlösung vollständig bedeckte Träger ab dem Beginn der Behandlung mit der Fixierlösung rotierend bewegt.

Das den Vinylacetatreaktor verlassende Reaktionsgemisch enthält Vinylacetat, nicht umgesetzte Essigsäure und Ethylen, geringe Mengen an nicht umgesetztem Sauerstoff sowie Inerte, beispielsweise Kohlendioxid und Stickstoff. Das Reaktionsgemisch wird in dem Vinylacetatreaktor nachgeschalteten Trenngefäßen in eine gasförmige Fraktion, die nicht umgesetztes Ethylen sowie einen Teil der Inerte enthält, und in eine flüssige Produktfraktion zerlegt, die nicht umgesetzte Essigsäure, Vinylacetat und andere verflüssigbare Reaktionsnebenprodukte enthält. Die gasförmige Fraktion wird in den Prozess zurückgeschleust und auch als Kreisgas bezeichnet. Aus der flüssigen Produktfraktion werden nach bekannten Verfahren, beispielsweise gemäß US-A-3,551,299 Vinylacetat und flüchtige Nebenprodukte, wie Acetaldehyd, Methyl- und Ethylacetat durch azeotrope Destillation mit Wasser abgetrennt, während im Destillationsrückstand Essigsäure anfällt. Vinylacetat wird anschließend nach an sich bekannten Verfahren, beispielsweise gemäß US-A-3,458,406, von Methyl- und Ethylacetat gereinigt. In Ullmann's encyclopädia of industrial chemistry, 5. Auflage 1996, Band 27, Seiten 423 bis 426 wird das Gasphasenverfahren zur Herstellung von Vinylacetat und die destillative Aufarbeitung des rohen Vinylacetats zusammenfassend dargestellt.

In der US 6,420,595 B1 wird ein Verfahren zur Echtzeit-Verfahrenssteuerung einer Vinylacetat Produktionsanlage beschrieben, bei der die Oxidation von Ethylen und Essigsäure als Parameter verwendet wird.

Für die sichere Prozeßführung ist das Einhalten der Zündgrenze im Kreisgas von großer Bedeutung. Die Zündgrenze hängt im wesentlichen von dem Essigsäuregehalt im Kreisgas ab und im Essigsäure gesättigten Zustand liegt die Zündgrenze höher als im Essigsäure freien Zustand. Daher ist die ausreichende Sättigung des Kreisgases mit Essigsäure für einen sicheren Betrieb des Vinylacetatprozesses von großer Bedeutung.

Bei der Sättigung des Kreisgases mit Essigsäure verwendet man sowohl frische Essigsäure als auch Essigsäure, die bei der Aufarbeitung der Reaktionsprodukte wiedergewonnen wird. Beispielsweise gewinnt man Essigsäure aus dem Destillationsrückstand bei der Vinylacetatabtrennung zurück.

Die Verwendung der aus dem Vinylacetatprozeß wiedergewonnenen Essigsäure, die auch als Rückessigsäure bezeichnet wird, bereitet für die Sättigung des Kreisgases jedoch Schwierigkeiten, da neben Essigsäure auch noch hochsiedende Verbindungen und Stoffe von geringer Flüchtigkeit in der Rückessigsäure vorhanden sind, die zu Polymerisationen neigen, beispielsweise Acetoxyvinylacetat. Die Anwesenheit dieser Verunreinigungen führt bei dem Sättigungsprozeß, bei dem man Rückessigsäure in Gegenwart von Kreisgas verdampft, zu Verschmutzungen in der Verdampfungseinrichtung und auf den dort vorhandenen Wärmeübertragungsflächen, mit dem Nachteil einer verminderten Wärmewirtschaftlichkeit bei der Essigsäuresättigung.

Ein Verfahren zur Entfernung der hochsiedenden Reaktionsnebenprodukte aus der im Kreislauf zu führenden Rückessigsäure, bevor diese wieder dem Vinylacetatreaktor zugeführt wird, ist aus der Deutschen Offenlegungsschrift 23 59 286 bekannt. Hierbei wird zunächst in einem Essigsäureverdampfer Ethylen haltiges Gas, beispielsweise Kreisgas, mit Rückessigsäure bei erhöhter Temperatur in Kontakt gebracht, so dass sich ein mit Essigsäure gesättigter Gasstrom bildet. Dieser Gasstrom wird anschließend auf den Boden einer Gegenstrom-Abtriebskolonne geleitet, dem die auf dem oberen Ende aufgegebene, zu reinigende Rückessigsäure entgegenströmt. In einer bevorzugten Ausgestaltung befindet sich über dem Abtriebsteil der Kolonne noch ein Rektifizier- oder Absorptionsabschnitt, auf dessen Kopf frische Essigsäure mit verhältnismäßig wenig Verunreinigungen gegeben wird. Das die Kolonne verlassende gasförmige Gemisch aus Essigsäure und Ethylen enthält nur noch geringe Mengen an hochsiedenden Reaktionsnebenprodukten aus der Rückessigsäure, es ist mit Essigsäure gesättigt und es kann dem Vinylacetatreaktor zugeführt werden. Frisches Ethylen kann dem Prozess vor oder nach dem Abtriebsvorgang zugesetzt werden.

Nach dem bekannten Verfahren lagern sich bei längerer Betriebszeit Verschmutzungen im Essigsäureverdampfer ab. Daher muss regelmäßig der Essigsäureverdampfer gereinigt werden und es müssen Anlagenteile durch Reserveaggregate ersetzt werden. Diese Reinigungsmaßnahme hat eine Unterbrechung des Produktionsprozesses zur Folge.

Darüber hinaus kommt es beim Durchleiten des mit Essigsäure gesättigten Kreisgases durch die Essigsäureverdampfereinrichtung zu einem Druckverlust. Als Folge muß man einen Druckabfall vor dem im Reaktor in Kauf nehmen, was sich wiederum nachteilig auf die Ausbeute an Vinylacetat auswirkt, da die Vinylacetatausbeute in signifikanter Weise vom Reaktionsdruck im Reaktor abhängt.

Es besteht daher der Bedarf an einem Verfahren, das eine ausreichende Sättigung des den Vinylacetatreaktor zugeführten Ethylen haltigen Gases unter Verwendung der aus dem Vinylacetatprozeß wiedergewonnenen Essigsäure sicherstellt und das die zuvor erwähnten Nachteile nicht aufweist.

Gegenstand der vorliegenden Erfindung ist daher ein Verfahren zur Sättigung Ethylen haltiger Gase mit Essigsäure, die einem Vinylacetatreaktor zugeführt werden, bei dem man die in dem Vinylacetatprozeß wiedergewonnene Essigsäure auf eine Kolonne gibt, in der über den Boden zugeführtes Ethylen haltiges Gas mit Essigsäure gesättigt wird, dadurch gekennzeichnet, dass die Kolonne eine Gegenstrom-Abtriebssektion und eine Rektifikationssektion aufweist, und wobei man die wiedergewonnene Essigsäure auf die Gegenstrom-Abtriebssektion und/oder auf die Rektifikationssektion aufgibt und wobei man frische Essigsäure auf die Rektifikatonssektion aufgibt und wobei man die der Kolonne entnommene Flüssigkeit in zwei Teilströme aufteilt und einen Teilstrom über eine Pumpe in die Kolonne unter Aufrechterhaltung eines zur ausreichenden Essigsäuresättigung erforderlichen Mindestumpumps zurückpumpt, wobei der Mindestumpump wenigstens die dreifache, vorzugsweise die siebenfache Menge der aufgegebenen wiedergewonnene Essigsäure beträgt und wobei dieser Teilstrom sicherheitstechnisch überwacht wird und vor Eintritt in die Kolonne in einem Wärmetauscher aufgeheizt wird, zu dem mindestens ein weiterer Wärmeaustauscher parallel angeordnet ist, und den anderen Teilstrom ausschleust.

Der für das erfindungsgemäße Verfahren charakteristische Mindestumpump besteht aus einer Entnahmeleitung für die am Kolonnenboden abfliessende Flüssigkeit, einer Trenneinrichtung zur Bildung von zwei Flüssigkeitsströmen, von denen ein Flüssigkeitsstrom ausgeschleust wird und der andere Flüssigkeitsstrom über eine Pumpe und einen Wärmeaustauscher, zu dem mindestens ein weiterer Wärmetauscher parallel angeordnet ist, wieder in die Kolonne, die man auch als Sättigungskolonne bezeichnet, zurückgeführt wird. In dem Mindestumpump werden übliche korrosionsbeständige Edelstähle verwendet.

Nach dem erfindungsgemäßen Verfahren muss ein Mindestumpump des in die Kolonne zurückzuführenden Teilstromes gewährleistet werden, um eine ausreichende Essigsäuresättigung des zugeführten Ethylen haltigen Gases zu erzielen. Aus sicherheitsstechnischen Gründen ist eine sichere Sättigung des zugeführten Ethylen haltigen Gases mit Essigsäure für die nachfolgende Einspeisung in den Vinylacetatreaktor von großer Bedeutung, da die Zündgrenze dieses dem Vinylacetatreaktor zuzuführenden Gasgemisches von
seinem Essigsäuregehalt abhängt und mit zunehmendem Essigsäuregehalt ansteigt. Um eine sichere Sättigung des der Sättigungskolonne zugeführten Ethylen haltigen Gases mit Essigsäure zu gewährleisten, ist ein ausreichender Mindestumpump erforderlich, der sicherheitstechnisch überwacht wird.

Die sicherheitstechnische Überwachung erfolgt durch mindestens zwei Messeinrichtungen zur Bestimmung von Durchflussmengen, die jeweils auf unterschiedlicher Gerätetechnik basieren. Die sicherheitstechnische Überwachung ist somit charakterisiert durch eine quantitative Größe, d.h. durch die Zahl der Messeinrichtungen, sowie durch eine qualitative Größe, d.h. durch die Prinzipien der angewandten Messverfahren. Die Messprinzipien zur Bestimmung von Durchflussmengen sind dem Fachmann an sich bekannt, beispielsweise Blendenmessung, Massendurchflussmessung, Wirbeldurchflussmessung, Ultraschallmessung oder Magnetisch-induktive Durchflussmessung (MID). Da die Überwachung der Durchflussmengen im Mindestumpump auf mindestens zwei Messeinrichtungen mit jeweils unterschiedlichen Messprinzipien beruht, ist selbst bei Ausfall einer Messeinrichtung noch ein sicherer Betrieb des Vinylacetatprozesses gewährleistet. Beispielsweise wird eine Messeinrichtung zur Bestimmung der Durchflussmengen nach der Ultraschallmethode, die andere Messeinrichtung nach der Wirbeldurchflussmethode betrieben.

Der erforderliche Mindestumpump kann aus der jeweiligen Betriebskonfiguration heraus abgeleitet werden und beträgt wenigstens die dreifache, vorzugsweise die siebenfache Menge der auf die Sättigungskolonne aufgegebenen, wiedergewonnenen Essigsäure.

In dem Mindestumpump ist ein Wärmetauscher eingebaut, um die Flüssigkeit vor Eintritt in die Sättigungskolonne auf eine solche Temperatur aufzuheizen, damit ausreichend Energie für die Essigsäureverdampfung in der Sättigungskolonne bereitgestellt wird.

Um eine ausreichende Wärmeübertragung für die Verdampfung der Essigsäure in der Sättigungskolonne zu gewährleisten, ist daher ein recht hoher Mindestumpump erforderlich.

Zu dem in dem Mindestumpump eingebauten Wärmetauscher ist wenigstens ein weiterer Wärmeaustauscher parallel eingebaut. Die Wärmetauscher werden gleichzeitig oder, vorzugsweise wechselseitig, betrieben und können zu Reinigungszwecken jeweils getrennt voneinander aus dem Sättigungsprozeß herausgenommen werden, ohne dass es zu einer Unterbrechung des gesamten Vinylacetatherstellprozesses kommt.

Das erfindungsgemäße Verfahren verzichtet auf den bekannten vorgeschalteten Essigsäureverdampfer und Verschmutzungsprobleme im Essigsäureverdampfer lassen sich umgehen und die zu ihrer Behebung erforderliche Betriebsunterbrechung läßt sich vermeiden. Durch die erfindungsgemäß in den Mindestumpump parallel eingebauten und jeweils getrennt austauschbaren Wärmetauscher kann der Sättigungsprozeß in der Sättigungskolonne kontinuierlich betrieben werden.

Ebenfalls kommt der am Essigsäureverdampfer anfallende Druckverlust nicht mehr zum Tragen, so dass ein höherer Druck im Vinylacetatreaktor aufrechterhalten werden kann, was sich wiederum vorteilhaft auf die Selektivität der Vinylacetatbildung und damit auf die Kapazität der Vinylacetatanlage auswirkt.

Das über den Boden in die Sättigungskolonne einzuleitende Ethylen kann rein sein oder mit anderen Gasen verdünnt sein, zum Beispiel mit Stickstoff. Vorzugsweise führt man jedoch denjenigen Ethylen haltigen Gasstrom zu, der aus dem den Vinylacetatreaktor verlassenden Reaktionsgemisch gewonnen wird und wieder in den Vinylacetatreaktor zurückgeführt wird. Im allgemeinen wird dieser zurückgewonnene Ethylen haltige Gasstrom auch Kreisgas genannt. Neben Ethylen enthält das Kreisgas noch Inerte, beispielsweise Stickstoff oder Kohlendioxid sowie geringe Mengen Sauerstoff. Üblicherweise wird diesem Kreisgas frisches Ethylen vor dem Eintritt in die Sättigungskolonne zugesetzt.

Die Sättigungskolonne besteht aus einer Gegenstrom-Abtriebssektion und einer Rektifikationssektion.

In einer bevorzugten Ausgestaltung des erfindungsgemäßen Verfahrens wird der Mindestumpump mit der wiedergewonnenen Essigsäure versetzt. Anschließend wird das Gemisch in dem oder den Wärmetauschern aufgeheizt und auf den Kopf der Gegenstrom-Abtriebssektion gegeben, dem das über den Kolonnenboden zugeführte Ethylen haltige Gasgemisch entgegenströmt, wobei es zu einer Beladung des Gasstromes mit Essigsäure kommt.

Es ist aber auch möglich, die wiedergewonnene Essigsäure an einer von der Aufgabestelle des Mindestumpumps getrennten Stelle auf die Sättigungskolonne zu geben, entweder in der Gegenstrom-Abtriebssektion oder auf die Rektifikationssektion. Wird die wiedergewonnene Essigsäure auf die Gegentrom-Abtriebssektion der Sättigungskolonne gegeben, empfiehlt es sich, die wiedergewonnene Essigsäure in die Kolonne hinein zu verdüsen.

Die wiedergewonnene Essigsäure enthält im allgemeinen etwa 5 Gew.-% Wasser und bis zu 1 Gew.-% Hochsieder, beispielsweise Ethylendiacetat. Der Rest auf 100 Gew-% ist Essigsäure.

Über der Gegenstrom-Abtriebssektion ist in der Sättigungskolonne noch eine Rektifikationssektion angeordnet, in dessen Kopf verhältnismäßig reine Essigsäure eingeleitet wird, die dem mit Essigsäure gesättigten Ethylen haltigen Gas entgegenströmt. Mit dieser Maßnahme werden noch weitere hochsiedende Verunreinigungen, die in der wiedergewonnenen Essigsäure enthalten sein können, aus dem Gasstrom entfernt. Dadurch vermeidet man, dass sich diese hochsiedenden Verunreinigungen bis in den mit Katalysator gefüllten Vinylacetatreaktor durchschleppen.

Die Sättigungskolonne wird im allgemeinen als Bodenkolonne ausgeführt. Die einzelnen Böden sind auf an sich bekannte Weise für einen guten Kontakt zwischen Flüssigkeit und Dampf ausgelegt. Gegebenenfalls kann man auch eine Füllkörperkolonne oder eine Reihe von einfachen, am Boden mit Gasverteilern versehenen Gefässen, beispielsweise Glockenböden, für den Sättigungsprozeß verwenden.

In der Gegenstrom-Abtriebssektion der Sättigungskolonne sollte mindestens ein Abtriebsboden, vorzugsweise mindestens zwei Abtriebsböden installiert sein. Ferner erzielt man besonders gute Ergebnisse, wenn mindestens etwa fünf, vorzugsweise mindestens acht Rektifizierböden in der Rektifikationssektion über den Abtriebsböden angeordnet sind. Dadurch können Hochsieder, die in den vom oberen Ende der Gegenstrom-Abtriebssektion ausströmenden Dämpfen enthalten sind, unter Rückfluß in die Sättigungskolonne zurückgespült werden.

Es ist besonders vorteilhaft, wenn die Rektifikationssektion der Sättigungskolonne so gebaut ist, dass die geringst mögliche Flüssigkeitsmenge mitgerissen wird, um einen Austrag an Hochsiedern mit dem dampfförmigen Gemisch aus Ethylen und Essigsäure, das die Sättigungskolonne über den Kopf der Rektifikationssektion verlässt, möglichst zu vermeiden. So ist es zum Beispiel vorteilhaft, in der Rektifikationssektion Siebböden zu verwenden, die so gebaut und angeordnet sind, dass möglichst wenig Flüssigkeit mitgerissen wird.

Die vom Boden der Sättigungskolonne abfließende Flüssigkeit wird in eine Trenneinrichtung in zwei Teilströme geteilt, von denen ein Teilstrom in den Mindestumpump zurückgeführt wird, während der andere Teilstrom aus dem Sättigungsprozeß ausgeschleust wird. Im allgemeinen beträgt das Gewichtsverhältnis von ausgeschleustem Teilstrom zu in den Mindestumpump zurückgeführten Teilstrom 1 : 10 bis 20, vorzugsweise 1 : 13 bis 16. Der ausgeschleuste Teilstrom kann einem Hochtemperatur-Dünnschichtverdampfer zugeführt werden, in dem Essigsäure als flüchtiger Bestandteil zurückgewonnen wird und mit an anderer Stelle des Vinylacetatprozesses zurückgewonnener Essigsäure vereinigt wird. Die im Hochtemperatur-Dünnschichtverdampfer anfallenden Hochsieder und Polymerisate werden aus dem Prozess ausgeschleust.

Temperatur und Druck in der Sättigungskolonne können innerhalb weiter Grenzen variieren; jedoch hält man den Druck gewöhnlich auf ungefähr der gleichen Höhe wie in dem Vinylacetatreaktor, zuzüglich einer ausreichenden Druckdifferenz, die erforderlich ist, um das Gasgemisch durch die Sättigungskolonne und durch die vor dem Vinylacetatreaktor angeordneten Anlagenteile zu treiben.

Da nach dem erfindungsgemäßen Sättigungsverfahren der separate Essigsäureverdampfer entfällt, wird ein im Vergleich zu dem Stand der Technik geringerer Druckabfall in der Sättigungsvorrichtung für das eingespeiste Kreisgas beobachtet, so dass zur Aufrechterhaltung der ausreichenden Druckdifferenz weniger Energie erfordert wird.

Ebenfalls wird über die im Mindestumpump parallel angeordneten Wärmetauscher Wärmeenergie in einem solchen Maße zugeführt, um die im Mindestumpump vorhandene Essigsäure soweit zu erhitzen, damit es zu einer ausreichenden Essigsäureverdampfung in der Sättigungskolonne kommt. Dadurch wird das der Sättigungskolonne zugeführte Ethylen haltige Gas bei den gewünschten Temperaturen mit Essigsäure gesättigt und mindestens ein Teil der über der Rektifikationssektion zugeführten frischen Essigsäure verdampft in den durch die Gegenstrom-Abtriebssektion geleiteten Gasstrom hinein.

Die Zusammensetzung der Flüssigkeit in dem Mindestumpump entspricht im wesentlichen der Zusammensetzung der wiedergewonnenen Essigsäure mit leicht erhöhten Anteilen an Hochsiedern. Ein zu hoher Gehalt an Hochsiedern in dem Mindestumpump ist zu vermeiden, um die Gefahr einer zu starken Verschmutzung der Wärmeübertragungsflächen an den Wärmetauschern in dem Mindestumpump zu reduzieren.

Bei einem zweckmäßigen Betrieb der Sättigungskolonne leitet man je Gewichtseinheit der über den Boden eingeleiteten Ethylen haltigen Gase soviel Essigsäure in Form von frischer Essigsäure, wiedergewonnener Essigsäure und Mindestumpump hinzu, dass das aus der Sättigungskolonne abgeführte, Ethylen haltige Gasgemisch bei seinem Sättigungspunkt einen Essigsäuregehalt von 10 bis 30 Gew.-% aufweist.

Die dem erfindungsgemäßen Sättigungsprozeß zugeführte Menge an wiedergewonnener Essigsäure wird in etwa konstant gehalten und frische Essigsäure wird an der Rektifikationssektion der Sättigungskolonne in mindestens einer solchen Menge zugesetzt, um eine ausreichende Waschwirkung zu erzielen. Dabei ist jedoch auch sicherzustellen, dass die über die Trenneinrichtung aus dem Sättigungsprozeß ausgeschleuste Flüssigkeitsmenge ausgeglichen wird, damit im Mindestumpump und in der Sättigungskolonne stets eine konstante Flüssigkeitsmenge vorliegt, und dass die über den Kopf der Sättigungskolonne in den Vinylacetatreaktor abgeführte Essigsäure stets ersetzt wird.

Das die Sättigungskolonne über den Kopf verlassende, mit Essigsäure gesättigte Ethylen haltige Gas wird vor dem Eintritt in den Vinylacetatreaktor mit einer für die Anlagenauslastung innerhalb der Betriebsparameter erforderlichen Sauerstoffmenge versetzt.

In der beigefügten Zeichnung wird beispielhaft eine schematische Ausführungsform des erfindungsgemäßen Verfahrens dargestellt.

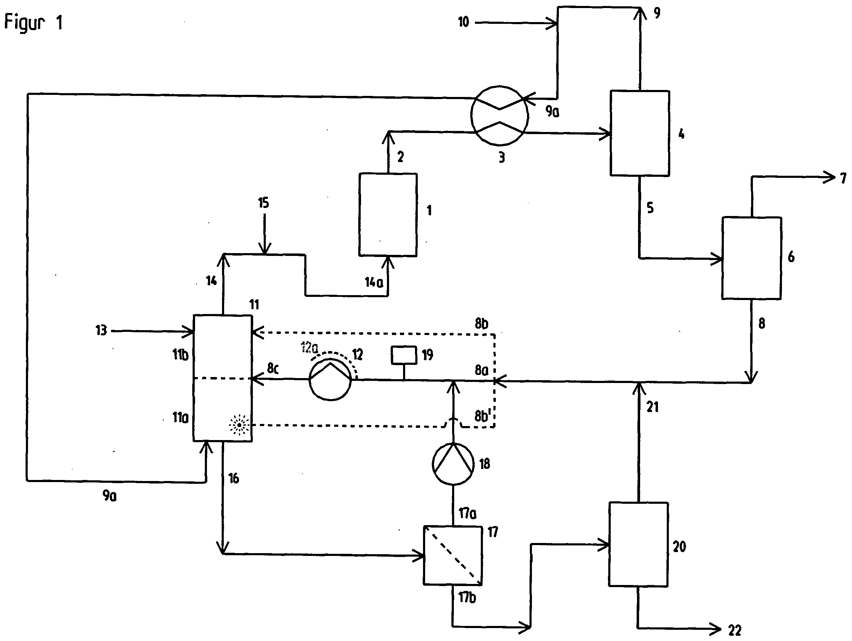

Nach dem in Figur 1 skizzierten Verfahrensablauf wird der den Vinylacetatreaktor 1 über die Leitung 2 verlassende Gasstrom zunächst in einem Gegenstromwärmetauscher 3 gekühlt und in einem Trenngefäß 4 in eine flüssige Phase und in eine gasförmige Phase getrennt. Die flüssige, rohes Vinylacetat enthaltende Phase wird über die Leitung 5 auf eine Destillationskolonne 6 gegeben, aus der über Kopf Vinylacetat über die Leitung 7 abgezogen wird, das dann nach weiteren bekannten Reinigungsverfahren, die in Figur 1 nicht dargestellt sind, aufgearbeitet wird. Aus dem Sumpf der Destillationskolonne 6 wird wiedergewonnene Essigsäure, die hochsiedende Verunreinigungen enthält, über die Leitung 8 der Sättigungskolonne 11 zugeführt.

Der dem Trenngefäß 4 am Kopf entnommene Gasstrom wird über die Leitung 9 abgezogen, mit frischem über die Leitung 10 zugeführtem Ethylen versetzt und in dem Gegenstromwärmetauscher 3 erwärmt, wobei das den Vinylacetatreaktor 1 verlassende Gasgemisch diesen über die Leitung 9a zurückzuführenden Gasstrom aufheizt. Der über die Leitung 9a zurückzuführende Ethylen haltige Gasstrom wird in die Sättigungskolonne 11 über den Boden eingeleitet. Über die Leitung 8 herangeführte wiedergewonnene Essigsäure wird über die Leitung 8a mit dem über die Leitung 17a herangeführten Mindestumpump vereinigt, in einem Wärmetauscher 12 aufgeheizt und über die Leitung 8c auf den Kopf der Gegenstrom-Abtriebssektion 11a der Sättigungskolonne 11 geführt, wobei die wiedergewonnene Essigsäure dem aufsteigenden Ethylen haltigen Gasstrom entgegenströmt und verdampft.

In einer weiteren Ausgestaltung des erfindungsgemäßen Verfahrens kann die wiedergewonnene Essigsäure aus Leitung 8 komplett oder teilweise über die Leitungen 8b und/oder 8b' (gestrichelt dargestellt) auf eine vom Mindestumpump getrennte Aufgabestelle auf die Sättigungskolonne gegeben werden. Bei dieser Ausführungsform des erfindungsgemäßen Verfahrens wird üblicherweise die wiedergewonnene Essigsäure auf die Rektifikationssektion gegeben. Bei der Aufgabe der wiedergewonnenen Essigsäure auf die Gegenstrom-Abtriebssektion 11a empfiehlt es sich, die wiedergewonnene Essigsäure in die Gegenstrom-Abtriebssektion 11a hinein zu verdüsen.

An die Gegenstrom-Abtriebssektion 11a schließt sich die Rektifikationssektion 11 b an, auf dessen Kopf über die Leitung 13 frische Essigsäure gegeben wird und in der weitere hochsiedende Verunreinigungen aus dem aufsteigenden mit Essigsäure gesättigten Ehylen haltigen Gasstrom gewaschen werden. Das über die Leitung 14 abgeführte mit Essigsäure gesättigte Gas wird mit Sauerstoff, der über die Leitung 15 herangeführt wird, versetzt und dann über die Leitung 14a dem Vinylacetatreaktor zugeführt.

Die vom Boden der Sättigungskolonne 11 abgezogene Flüssigkeit wird über die Leitung 16 einer Trenneinrichtung 17 zugeführt, in der ein Flüssigkeitsteilstrom abgetrennt und mit Hilfe der Pumpe 18 über die Leitung 17a auf die Leitung 8a gepumpt wird. In der Leitung 8c werden die Stoffströme aus der Leitung 17a und 8a vereinigt, in dem Wärmetauscher 12 aufgeheizt und anschließend auf den Kopf der Gegenstrom-Abtriebssektion 11a der Sättigungskolonne 11 gegeben.

Führt man in einer anderen Ausgestaltung der Erfindung die wiedergewonnene Essigsäure ausschließlich über die Leitungen 8b und/oder 8b' der Sättigungskolonne 11 zu, so wird nur der über die Leitung 17a herangeführte Mindestumpump nach Durchlaufen durch den Wärmetauscher 12 über die Leitung 8c auf die Sättigungskolonne gegeben.

Zur sicherheitstechnischen Überwachung des Mindestumpumps sind in dem Mindestumpump mindestens zwei Einrichtungen 19 zur Messung von Durchflußmengen eingebaut, die jeweils auf unterschiedlichen Gerätetechniken basieren.

Der andere, in der Trenneinrichtung 17 anfallende Flüssigkeitsteilstrom wird über die Leitung 17b abgeführt und beispielsweise einem Dünnschicht-Verdampfer 20 zugeführt, in dem der Kopfabzug 21, enthaltend Essigsäure, mit der über Leitung 8 herangeführten wiedergewonnenen Essigsäure vereinigt wird, während der hochsiedende Rückstand über die Leitung 22 aus dem Prozess ausgeschleust wird.

In dem Mindestumpump ist zudem wenigstens ein weiterer parallel angeordneter Wärmeaustauscher 12a eingebaut (gestrichelt dargestellt). Die Wärmetauscher werden vorzugsweise wechselseitig betrieben und können zu Reinigungszwecken separat entfernt werden, ohne den Sättigungsprozeß zu unterbrechen.

Für einen sicheren Betrieb der Vinylacetatreaktion muß die ausreichende Sättigung des Reaktionsgases mit Essigsäure sichergestellt werden. Dies wird dadurch erzielt, dass ein Mindestumpump aufrechterhalten wird, der sicherheitstechnisch überwacht wird.

## Patentansprüche

1. Verfahren zur Sättigung Ethylen haltiger Gase mit Essigsäure, die einem Vinylacetatreaktor zugeführt werden, bei dem man die in dem Vinylacetatprozess wiedergewonnene Essigsäure auf eine Kolonne gibt, in der über den Boden zugeführtes Ethylen haltiges Gas mit Essigsäure gesättigt wird, **dadurch gekennzeichnet, dass** die Kolonne eine Gegenstrom-Abtriebssektion und eine Rektifikationssektion aufweist, und wobei man die wiedergewonnene Essigsäure auf die Gegenstrom-Abtriebssektion und/oder auf die Rektifikationssektion aufgibt und wobei man frische Essigsäure auf die Rektifikatonssektion aufgibt und wobei man die der Kolonne entnommene Flüssigkeit in zwei Teilströme aufteilt und einen Teilstrom über eine Pumpe in die Kolonne unter Aufrechterhaltung eines zur ausreichenden Essigsäuresättigung erforderlichen Mindestumpumps zurückpumpt, wobei der Mindestumpump wenigstens die dreifache, vorzugsweise die siebenfache Menge der aufgegebenen wiedergewonnene Essigsäure beträgt und wobei dieser Teilstrom sicherheitstechnisch überwacht wird und vor Eintritt in die Kolonne in einem Wärmetauscher aufgeheizt wird, zu dem mindestens ein weiterer Wärmetauscher parallel angeordnet ist, und den anderen Teilstrom ausschleust.

2. Verfahren nach Anspruch 1, **dadurch gekennzeichnet, dass** die Wärmetauscher wechselseitig betrieben werden.

3. Verfahren nach einem oder mehreren der Ansprüche 1 bis 2, **dadurch gekennzeichnet, dass** man den Mindestumpump auf den oberen Teil der Gegenstrom-Abtriebssektion aufgibt.

4. Verfahren nach Anspruch 3, **dadurch gekennzeichnet, dass** man den Mindestumpump auf den Kopf der Gegenstrom-Abtriebssektion aufgibt.

5. Verfahren nach einem oder mehreren der Ansprüche 1 bis 4, **dadurch gekennzeichnet, dass** der Mindestumpump mit der gesamten Menge oder mit einem Teil der wiedergewonnenen Essigsäure vor Aufgabe auf die Kolonne vereinigt wird.

6. Verfahren nach einem oder mehreren der Ansprüche 1 bis 5, **dadurch gekennzeichnet, dass** frische Essigsäure auf den oberen Teil, vorzugsweise auf den Kopf, der Rektifikationssektion gegeben wird.

7. Verfahren nach einem oder mehreren der Ansprüche 1 bis 6, **dadurch gekennzeichnet, dass** die Kolonne als Bodenkolonne, Füllkörperkolonne oder Glockenbodenkolonne ausgelegt ist.

8. Verfahren nach einem oder mehreren der Ansprüche 1 bis 7, **dadurch gekennzeichnet, dass** in der Gegenstrom-Abtriebssektion mindestens ein, vorzugsweise mindestens zwei Abtriebsböden angeordnet sind und dass in der Rektifikationssektion mindestens fünf, vorzugsweise mindestens acht Rektifizierböden angeordnet sind.

9. Verfahren nach einem oder mehreren der Ansprüche 1 bis 8, **dadurch gekennzeichnet, dass** das Gewichtsverhältnis von ausgeschleustem Teilstrom zu in den Mindestumpump zurückgeführten Teilstrom 1: 10 bis 20, vorzugsweise 1: 13 bis 16, beträgt.

10. Verfahren nach einem oder mehreren der Ansprüche 1 bis 9, **dadurch gekennzeichnet, dass** je Gewichtseinheit des zugeführten Ethylen haltigen Gases soviel Essigsäure in Form von frischer Essigsäure, wiedergewonnener Essigsäure und Mindestumpump hinzugeführt werden, dass das aus der Kolonne abgeführte Ethylen haltige Gasgemisch bei seinem Sättigungspunkt einen Essigsäuregehalt von 10 bis 30 Gew.-% aufweist.

## Claims

1. A method for saturating ethylene-containing gases with acetic acid which are fed to a vinyl acetate reactor, wherein the acetic acid recovered in the vinyl acetate process is charged to a column in which ethylene-containing gas fed via the bottom is saturated with acetic acid, **characterized in that** the column has a countercurrent stripping section and a rectification section, and wherein the recovered acetic acid is charged to the countercurrent stripping section and/or to the rectification section and wherein fresh acetic acid is charged to the rectification section and wherein the liquid taken off the column is divided into two substreams and one substream is pumped back into the column via a pump while maintaining a minimum pumped circulation required for sufficient saturation with acetic acid, wherein the minimum pumped circulation amounts to at least three times, preferably seven times, the amount of the charged recovered acetic acid and wherein said substream is monitored in terms of safety and before entering the column is heated in a heat exchanger with which at least one further heat exchanger is arranged in parallel, and the other substream is discharged.

2. The method according to claim 1, **characterized in that** the heat exchangers are operated reciprocally.

3. The method according to one or more of claims 1 to 2, **characterized in that** the minimum pumped circulation is charged to the upper part of the countercurrent stripping section.

4. The method according to claim 3, **characterized in that** the minimum pumped circulation is charged to the top of the countercurrent stripping section.

5. The method according to one or more of claims 1 to 4, **characterized in that** the minimum pumped circulation is combined with the total amount or with a part of the recovered acetic acid before being charged to the column.

6. The method according to one or more of claims 1 to 5, **characterized in that** fresh acetic acid is charged to the upper part, preferably to the top, of the rectification section.

7. The method according to one or more of claims 1 to 6, **characterized in that** the column is configured as a tray column, a packed column or a bubble-tray column.

8. The method according to one or more of claims 1 to 7, **characterized in that** at least one, preferably at least two, stripping trays are arranged in the countercurrent stripping section and that at least five, preferably at least eight, rectification trays are arranged in the rectification section.

9. The method according to one or more of claims 1 to 8, **characterized in that** the weight ratio of discharged substream to substream recirculated to the minimum pumped circulation amounts to 1:10 to 20, preferably 1:13 to 16.

10. The method according to one or more of claims 1 to 9, **characterized in that** acetic acid in the form of fresh acetic acid, recovered acetic acid and minimum pumped circulation is added in an amount per unit weight of the fed ethylene-containing gas such that the ethylene-containing gas mixture removed from the column has an acetic acid content of 10 to 30% by weight at its saturation point.

## Revendications

1. Procédé pour saturer d'acide acétique des gaz contenant de l'éthylène, qui sont amenés à un réacteur de production d'acétate de vinyle, dans lequel on place sur une colonne l'acide acétique récupéré dans le procédé de production d'acétate de vinyle, colonne dans laquelle le gaz contenant de l'éthylène, amené au-dessus des plateaux, est saturé en acide acétique, **caractérisé en ce que** la colonne comprend une section d'entraînement à contre-courant et une section de rectification, et dans lequel on introduit dans la section d'entraînement à contre-courant et/ou dans la section de rectification l'acide acétique récupéré, et dans lequel on introduit sur la section de rectification de l'acide acétique frais, et dans lequel on subdivise en deux courants partiels le liquide prélevé de la colonne, et on renvoie dans la colonne en recirculation un courant partiel, par l'intermédiaire d'une pompe, en maintenant une recirculation minimale, afin d'obtenir une saturation suffisante en acide acétique, la recirculation minimale étant au moins trois fois égale et de préférence sept fois à la quantité de l'acide acétique récupéré introduit, et ce courant partiel faisant l'objet d'une surveillance du point de vue des règlements de sécurité, et étant chauffé, avant de pénétrer dans la colonne, dans un échangeur de chaleur, au moins un échangeur de chaleur supplémentaire étant disposé en parallèle à ce dernier, et évacuant l'autre courant partiel.

2. Procédé selon la revendication 1, **caractérisé en ce que** les échangeurs de chaleur sont exploités en alternance.

3. Procédé selon l'une ou plusieurs des revendications 1 à 2, **caractérisé en ce qu'**on introduit la recirculation minimale dans la partie supérieure de la section d'entraînazud den Kopfement à contre-courant.

4. Procédé selon la revendication 3, **caractérisé en ce qu'**on introduit la recirculation minimale en tête de la section d'entraînement à contre-courant.

5. Procédé selon l'une ou plusieurs des revendications 1 à 4, **caractérisé en ce que** la recirculation minimale est réunie à la quantité totale ou partielle de l'acide acétique récupéré, avant introduction dans la colonne.

6. Procédé selon l'une ou plusieurs des revendications 1 à 5, **caractérisé en ce que** de l'acide acétique frais est introduit dans la partie supérieure, de préférence en tête, de la section de rectification.

7. Procédé selon l'une ou plusieurs des revendications 1 à 6, **caractérisé en ce que** la colonne est conçue comme une colonne à plateaux, une colonne à garnissage ou une colonne à plateaux de barbotage.

8. Procédé selon l'une ou plusieurs des revendications 1 à 7, **caractérisé en ce qu'**au moins un, de préférence au moins deux plateaux d'entraînemement sont disposés dans la section d'entraînement à contre-courant, et qu'au moins cinq, de préférence au moins huit plateaux de rectification sont disposés dans la section de rectification.

9. Procédé selon l'une ou plusieurs des revendications 1 à 8, **caractérisé en ce que** le rapport pondéral, entre le courant partiel évacué et le courant partiel renvoyé dans la recirculation minimale, est de 1:10 à 20, de préférence de 1:13 à 16.

10. Procédé selon l'une ou plusieurs des revendications 1 à 9, **caractérisé en ce que**, par unité de poids de gaz contenant de l'éthylène, on amène sous forme d'acide acétique frais, d'acide acétique récupéré et de recirculation minimale, une quantité d'acide acétique suffisante pour que le mélange gazeux contenant de l'éthylène, évacué de la colonne, présente en son point de saturation une teneur en acide acétique de 10 à 30 % de son poids.
